(19) Europäisches Patentamt
European Patent Office
Office européen des brevets

(11) Veröffentlichungsnummer: **0 334 809 B1**

(12) # EUROPÄISCHE PATENTSCHRIFT

(45) Veröffentlichungstag der Patentschrift: **15.06.94**

(21) Anmeldenummer: **89810198.5**

(22) Anmeldetag: **14.03.89**

(51) Int. Cl.5: **C07D 405/12**, C07D 401/12, C07D 417/12, C07D 413/12, C07D 409/12, A01N 43/40, A01N 43/50, A01N 43/653, A01N 43/74, A01N 43/80, A01N 43/82

(54) **Mittel zum Schutz von Pflanzen gegen Krankheiten.**

(30) Priorität: **21.03.88 CH 1068/88**

(43) Veröffentlichungstag der Anmeldung:
**27.09.89 Patentblatt 89/39**

(45) Bekanntmachung des Hinweises auf die Patenterteilung:
**15.06.94 Patentblatt 94/24**

(84) Benannte Vertragsstaaten:
**CH DE ES FR GB IT LI NL**

(56) Entgegenhaltungen:
**EP-A- 0 013 360**
**EP-A- 0 246 507**
**US-A- 4 137 067**

**PATENT ABSTRACTS OF JAPAN Band 12, Nr, 382 ( C-535 )(3229), 12. Oktober 1988; & JP - A - 63 130583 (NIPPON KAYAKU CO. LTD.) 02.06.1988**

(73) Patentinhaber: **CIBA-GEIGY AG**
**Klybeckstrasse 141**
**CH-4002 Basel(CH)**

(72) Erfinder: **Eckhardt, Wolfgang Dr.**
**Breslauerstrasse 14**
**D-7850 Lörrach(DE)**
Erfinder: **Meyer, Alfred, Dr.**
**St. Galler-Ring 220**
**CH-4054 Basel(CH)**
Erfinder: **Kunz, Walter, Dr.**
**Buchenstrasse 9**
**CH-4104 Oberwil(CH)**

**EP 0 334 809 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Isonicotinsäureamide und Isonicotinsäurehydrazide der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

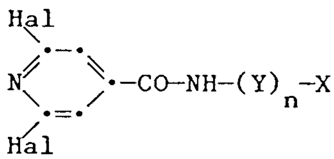

(I)

in welcher bedeuten:

Hal unabhängig voneinander Fluor, Chlor, Brom oder Jod, vorzugsweise Fluor, Chlor oder Brom;
Y NH;
n 1 oder 0;
X ein 5-gliedriger gesättigter oder ungesättigter unsubstituierter oder mit $C_1$-$C_8$-Alkyl, Halogen, Trifluormethyl, Cyano oder Nitro substituierter Heterocyclus mit 1-3 Heteroatomen, wie N, O oder S, wobei der Heterocyclus zusätzlich eine Oxo- oder Thionogruppe enthalten kann.

Alkyl selbst oder als Bestandteil eines anderen Substituenten bedeutet geradkettige oder verzweigte Alkyle. Sie stellen je nach Zahl der angegebenen Kohlenstoffatome beispielsweise folgende Gruppen dar: Methyl, Ethyl sowie die Isomeren von Propyl, Butyl, Pentyl, Hexyl, Heptyl oder Octyl, wie z.B. Isopropyl, Isobutyl, tert.-Butyl, sek.-Butyl oder Isopentyl.

Unter 5-gliedrigen Heterocyclen sind beispielsweise zu verstehen: Furan, Tetrahydrofuran, Thiophen, Pyrrol, Imidazol, Imidazolin, Pyrazol, Pyrazolin, Triazol, Oxazol, Isoxazol, Thiazol, Isothiazol, Thiadiazol, Oxadiazol, Thiazolin oder Oxazolin.

Als Bestandteil von Verbindungen der Formel I sind die heterocyclischen Reste z.B. gebunden als: Furan-2-yl, Furan-3-yl, Thiophen-2-yl, Thiophen-3-yl, Imidazol-2-yl, 4,5-Imidazolin-2-yl, Pyrazol-3-yl, Pyrazolin-3-yl, 1,2,4-Triazol-3-yl, 1,2,4-Triazol-4-yl, Oxazol-2-yl, Isoxazol-5-yl, Isoxazol-3-yl, Thiazol-2-yl, Isothiazol-3-yl, Isothiazol-5-yl, 1,3,4-Thiadiazol-2-yl, 1,3,4-Thiadiazol-3-yl, 1,3,4-Oxadiazol-3-yl oder 1,2,4-Oxadiazol-3-yl sowie ferner 3-Thiono-1,2-dithia-4-azol-5-yl, 2-Thiono-thiazolin-4-on-3-yl oder 2-Oxy-4,5-dihydrooxazol-3-yl.

Wegen hervorragender biologischer Aktivität sind von den Verbindungen der Formel I folgende Gruppen bevorzugt:

1. Verbindungen gemäss Anspruch 1 der Formel 1b

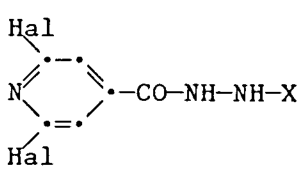

(Ib)

worin bedeuten:

Hal unabhängig voneinander Fluor, Chlor oder Brom;
X ein wie unter Formel I angegebener Heterocyclus.

2. Verbindungen der Formel Ic

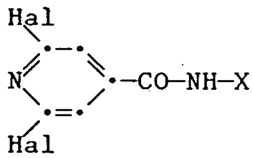

(Ic)

worin bedeuten:

Hal unabhängig voneinander Fluor, Chlor oder Brom;

X ein wie unter Formel I angegebener Heterocyclus.

Es sind diejenigen Wirkstoffe der Formel I bevorzugt, die folgende Substituenten oder Kombinationen dieser Substituenten untereinander aufweisen:

1.1. Verbindungen der Formel Ib, worin bedeuten:

Hal Chlor oder Brom;

X Furan-2-yl, 1,2,4-Triazol-3-yl, Thiazol-2-yl, Thiazol-5-yl, Isothiazol-3-yl, Oxazol-2-yl, Isoxazol-3-yl, Isoxazol-5-yl, 1,3,4-Thiadiazol-2-yl, 1,2,4-Thiadiazol-3-yl oder 3-Thiono-1,2-dithia-4-azol-5-yl.

1.2. Verbindungen der Formel Ic, worin bedeuten:

Hal Chlor oder Brom;

X Furan-2-yl, 1,2,4-Triazol-3-yl, Thiazol-2-yl, Thiazol-5-yl, Isothiazol-3-yl, Oxazol-2-yl, Isoxazol-3-yl, Isoxazol-5-yl, 1,3,4-Thiadiazol-2-yl, 1,2,4-Thiadiazol-3-yl oder 3-Thiono-1,2-dithia-4-azol-5-yl.

Ferner sind besonders bevorzugt die Verbindungen der Formeln Ib und Ic, in denen Hal Chlor bedeutet und X die unter den genannten Formeln angegebenen Bedeutungen darstellt.

Von den vorstehend aufgeführten Verbindungsgruppen sind insbesondere die Verbindungen der Formel Ic bevorzugt.

Die folgenden Verbindungen zeichnen sich durch besonders vorteilhafte pflanzenschützende Eigenschaften aus:

3-Methylisoxazol-5-(2,6-dichlorisonicotinsäure)-amid,
5-Methylisoxazol-3-(2,6-dichlorisonicotinsäure)-amid,
α-Methylfurfuryl-2,6-dichlorisonicotinsäureamid;
3-Methylisothiazol-5-(2,6-dichlorisonicotinsäure)-amid.

2,6-Dihaloisonicotinsäure-Derivate sind bereits teilweise bekannt. So sind in der britischen Patentschrift Nr. 923,387 2,6-Dihaloisonicotinsäurederivate, z.B. aliphatische Amide als Herbizide beschrieben. Ferner sind in der USA-Patentschrift No. 4,137,067 und in der kanadischen Patentschrift No. 1,072,443 2,6-Dichlorisonicotinsäurehydrazide zur Bekämpfung phytopathogener Mikroorganismen offenbart. Darüber hinaus sind 2,6-Dihaloisonicotinsäure-Derivate als Tuberkulostatika bekannt geworden (vgl. Acta Fac. Pharm. Brun. Bratislav. 4, 65-66 [1962]; Chem. Abstr. Vol 57, 1962, 4769b). Ferner sind N-Cyanoalkylisonicotinsäureamide als Fungizide oder Bakterizide in der EP-A-246507 offenbart.

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mikroorganismen mittels Blattapplikation (direkte Wirkung) oder Bodenapplikation (systemische Wirkung) als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung, kann ein breit

gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I zueigene systemische Aktivität, dass sich der Schutzeffekt auch auf zuwachsende Pflanzenteile der behandelten Pflanzen erstreckt.

Die allgemein pflanzenschützende der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizoctonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:
Pilze, wie z.B. Oomyceten (z.B. Plasmopara viticola, Phytophthora infestans, Peronospora tabacina, Pseudoperonospora), Fungi imperfecti (z.B. Colletotrichum lagenarium, Piricularia oryzae, Cercospora nicotinae), Ascomyceten (z.B. Venturia inaequalis);
Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen aus unterschiedlichen Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomate, Weizen, Gerste, Birne und Apfel.

Die Verbindungen der Formel I werden aus 2,6-Dihalogen-isonicotinsäurehalogeniden, -anhydriden oder -azoliden, vorzugsweise aus 2,6-Dihalogenisonicotinsäurehalogeniden oder besonders bevorzugt aus Dihalogenisonicotinsäurechloriden, gewonnen.

Die Verbindungen der Formel I werden hergestellt, indem man ein Isonicotinsäurehalogenid der Formel II

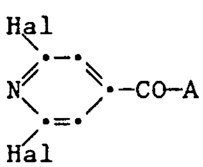 (II)

mit einem Amin der Formel III

$H_2N\text{-}(Y)_n\text{-}X$ (III)

in Gegenwart einer Base in einem inerten Lösungsmittel oder ohne Lösungsmittel bei Temperaturen von -20° bis 150°C, vorzugsweise 0° bis 80°C, umsetzt, wobei Hal Halogen, A Halogen, einen Acyloxyrest der Formel

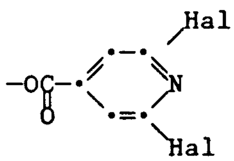

,

Imidazolyl oder Triazolyl bedeuten und X, Y und n die unter der Formel I angegebenen Bedeutungen besitzen.

Als Basen zur Säurebindung im vorgängig beschriebenen Verfahren kommen organische und anorganische Basen in Betracht, z.B. tertiäre Amine wie Trialkylamine (Trimethylamin, Triethylamin, Tripropylamin usw.), Pyridin, Pyridinbasen (4-Dimethylaminopyridin, 4-Pyrrolidylaminopyridin usw.), und Alkoholate wie z.B. Kaliumtert.-Butylat, Oxide und Hydroxide, Carbonate und Hydrogencarbonate von Alkali- und Erdalkalimetallen sowie Alkaliacetate.

In dem Verfahren werden als Reaktionsmedien in Anpassung an die jeweiligen Reaktionsbedingungen geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon; sowie Gemisch solcher Lösungsmittel untereinander.

Umsetzungsreaktionen von Säurehalogeniden bzw. -anhydriden mit Aminen sind in Houben-Weyl Bd. 18, S. 655 beschrieben, während über Umamidierung in Angew. Chemie 1962, S. 413/412 berichtet wird.

Die Herstellung der Ausgangsstoffe wie Säurehalogenide und -anhydride sowie Heterocyclen ist dem Fachmann geläufig und aus der Fachliteratur bekannt [z.B. Houben-Weyl 5/3, S. 925; Chemistry of Heterocyclic Compounds, Arnold Weisenberger und Edward C. Taylor, Vol. 4 (1952), Vol. 6 (1953), Vol. 34 (1979), Vol. 37 (1981), Vol. 44 (1985), Vol. 45 (1986)].

Die im Rahmen der Erfindung zur Anwendung gelangenden Mittel zum Schutz von Pflanzen gegen Krankheiten welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonders Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere

bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, und Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungsbeispiel

Beispiel 1.1: Herstellung von 5-Methylisoxazol-3-(2,6-dichlorisonicotinsäure)-amid

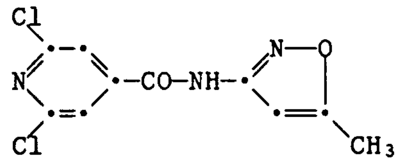

3 g (0,03 Mol) 3-Amino-5-methylisoxazol und 3 g (0,03 Mol) Triethylamin wurden in 70 ml Dichlormethan gelöst und bei 0°-5°C mit einer Lösung von 2,6-Dichlorisonicotinsäurechlorid in 30 ml Dichlormethan tropfenweise versetzt. Das Reaktionsgemisch wurde anschliessend über Nacht bei Raumtemperatur gerührt, dann auf 500 ml Wasser gegossen und das Produkt abfiltriert und getrocknet. Man erhält so 7 g (86 % d. Th.) Produkt vom Smp. (Zers.) > 270°C.

Gemäss der vorbeschriebenen Herstellung werden die nachfolgend aufgeführten Verbindungen erhalten.

Tabelle 1

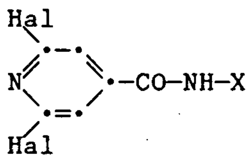

| Nr. | Hal | X | Physik. Daten |
|---|---|---|---|
| 1.1 | Cl | 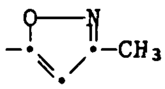 | Smp. 225°C (Zers.) |
| 1.2 | Br | 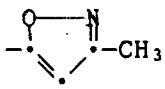 | Smp. 231-232°C |
| 1.3 | F | 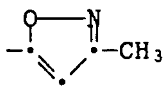 | |
| 1.4 | Cl | 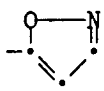 | |
| 1.5 | Br | 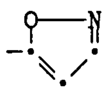 | |
| 1.6 | Cl | 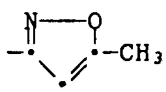 | Smp. 270°C (Zers.) |
| 1.7 | Br | 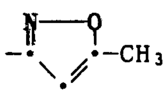 | Smp. 273-274°C |
| 1.8 | Cl | 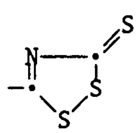 | Smp. 85°C (Zers.) |
| 1.9 | Br | 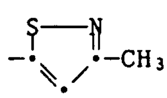 | |
| 1.10 | Cl | 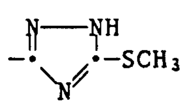 | |
| 1.11 | Cl | 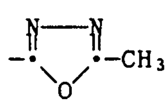 | |

Tabelle 1 (Fortsetzung)

| Nr. | Hal | X | Physik. Daten |
|---|---|---|---|
| 1.12 | Br | 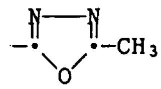 | |
| 1.13 | Cl | 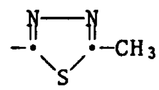 | Smp. 308-310°C |
| 1.14 | Br | 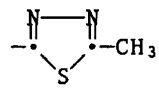 | |
| 1.15 | Cl | 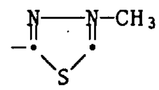 | |
| 1.16 | Br | 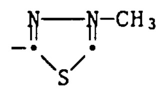 | |
| 1.17 | Cl | 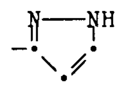 | Smp. 208-210°C |
| 1.18 | Cl | 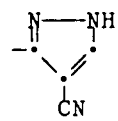 | |
| 1.19 | Cl | 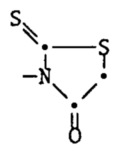 | Smp. 214-216°C |
| 1.20 | Cl | 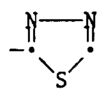 | Smp. > 305°C |
| 1.21 | Br | 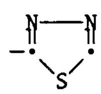 | |
| 1.22 | Cl | 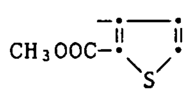 | |
| 1.23 | Cl | 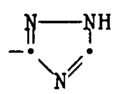 | Smp. 302-309°C |
| 1.24 | Cl | 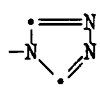 | Smp. 237-239°C |

Tabelle 1 (Fortsetzung)

| Nr. | Hal | X | Physik. Daten |
|---|---|---|---|
| 1.25 | Cl | 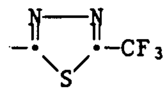 | Smp. 194-196°C |
| 1.26 | Br | 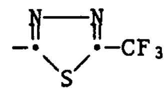 | |
| 1.27 | Cl | 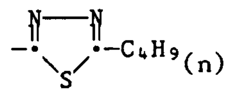 | Smp. 182-184°C |
| 1.28 | Cl | 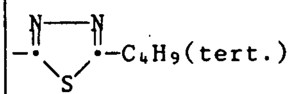 | Smp. 232-234°C |
| 1.29 | Cl | 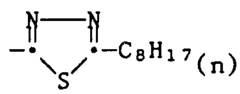 | |
| 1.30 | Cl | 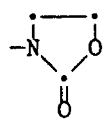 | Smp. 215-218°C |
| 1.31 | Cl | 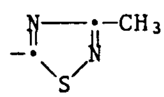 | Smp. 189-191°C |
| 1.32 | Cl | 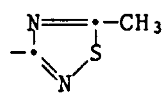 | Smp. 112-115°C |
| 1.33 | Cl | 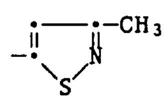 | Smp. 225-228°C |
| 1.34 | Cl | 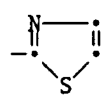 | Smp. 253-255°C |
| 1.35 | Cl | 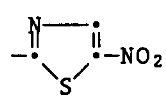 | Smp. 193-196°C |
| 1.36 | Cl | 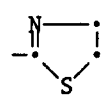 | Smp. 208-210°C |
| 1.37 | F | 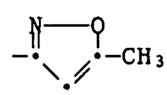 | |

Tabelle 1 (Fortsetzung)

| Nr. | Hal | X | Physik. Daten |
|---|---|---|---|
| 1.38 | Br | 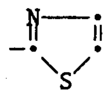 | Smp. 239-241°C |
| 1.39 | J | 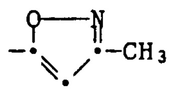 | Smp. 248-250°C |
| 1.40 | J | 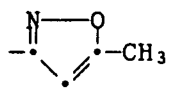 | |

Tabelle 2

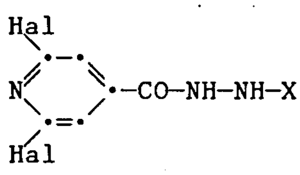

| Nr. | Hal | X | Physik. Daten |
|---|---|---|---|
| 3.1 | Cl | 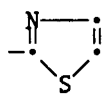 | |
| 3.2 | Br | 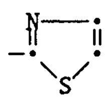 | |
| 3.3 | Cl | 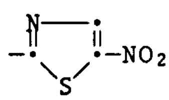 | |
| 3.4 | Cl | 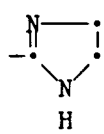 | |
| 3.5 | Cl | 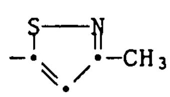 | |
| 3.6 | Br | 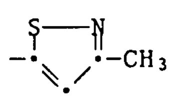 | |
| 3.7 | Cl | 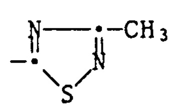 | Smp. 229-231°C |

2. Formulierungsbeispiele für Wirkstoffe der Formel I (% = Gewichtsprozent)

2.1 Spritzpulver

| | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

2.2 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

2.3 Stäubemittel

| | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

2.4 Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat

| | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm).

Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C bis 23 °C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 oder 20 ppm bezogen auf das Bodenvolumen).

Nach 48 Stunden werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22 °C weitergeführt.

Die Beurteilung der Schutzwirkung erfolgt aufgrund des Pilzbefalls 7-8 Tage nach der Infektion.

c) Gurkenpflanzen werden nach 2-wöchiger Anzucht mit einer aus Spritzpulver des Wirkstoffes hergestellten Spritzbrühe besprüht (Konzentration: 200 ppm).

Nach 3 Wochen werden die Pflanzen mit einer Sporensuspension ($1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchigkeit und bei 22° bis 23 °C weitergeführt.

Die Beurteilung der Schutzwirkung erfolgt aufgrund des Pilzbefalls 7-8 Tage nach der Infektion.

Verbindungen aus den Tabellen 1 und 2 zeigten in den Tests (a) und (b) gute Wirkung. So reduzierten z.B. die Verbindungen 1.1, 1.2, 1.6, 1.7, 1.8 und 1.23 den Pilzbefall auf 0 bis 20 %. Unbehandelte jedoch infizierte Kontrollpflanzen wiesen dagegen einen Colletotrichum-Befall von 100 % auf.

Beispiel 3.2: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

b) Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20 °C werden die infizierten Pflanzen in einem Gewächshaus bei ca. 22 °C aufgestellt. Die Beurteilung der Rostpustelnentwicklung erfolgt 12 Tage nach der Infektion.

Verbindungen aus den Tabellen 1 und 2 zeigten gegen Puccinia-Pilze eine gute Wirkung. So reduzierten z.B. die Verbindungen 1.1, 1.2, 1.30, und 3.7 den Pilzbefall auf 0 bis 20 %. Unbehandelte jedoch infizierte Kontrollpflanzen wiesen dagegen einen Puccinia-Befall von 100 % auf.

Beispiel 3.3: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgte nach einer Inkubation der infizierten Pflanzen während 5 Tagen bei 9O-100 % relativer Luftfeuchtigkeit und 20 °C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1 und 2 zeigten gegen den Phytophthora-Pilz eine gute Schutzwirkung. So reduzierten z.B. die Verbindungen 1.13, 1.17, 1.20 und 1.32 den Pilzbefall auf 0 bis 20 %. Unbehandelte jedoch infizierte Kontrollpflanzen wiesen dagegen einen 100 %igen Phytophthora-Befall auf.

Beispiel 3.4: Wirkung gegen Cercospora arachidicola auf Erdnusspflanzen

10-15 cm hohe Erdnusspflanzen werden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen werden während 72 Stunden bei ca. 21 °C und hoher Luftfeuchtigkeit inkubiert und anschliessend bis zum Auftreten der typischen Blattflecken in einem Gewächshaus aufgestellt.

Die Beurteilung der fungiziden Wirkung erfolgt 12 Tage nach der Infektion basierend auf Anzahl und Grösse der auftretenden Flecken.

Im Vergleich zu unbehandelten, jedoch infizierten Kontrollpflanzen (Anzahl und Grösse der Flecken = 100 %), zeigten Erdnusspflanzen, die mit Wirkstoffen aus den Tabellen 1 und 2 behandelt wurden, einen reduzierten Cercospora-Befall.

Beispiel 3.5: Wirkung gegen Plasmopara viticola auf Reben

a) Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20 °C wird der Pilzbefall beurteilt.

b) Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20 °C werden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages werden die behandelten Pflanzen wieder in die Feuchtkammer gebracht. Die Beurteilung des Pilzbefalls

erfolgt 6 Tage nach der Infektion.

Verbindungen aus den Tabellen 1 und 2 zeigten gegen Plasmopara viticola eine gute Schutzwirkung, so reduzierte z.B. die Verbindung 1.8 den Pilzbefall auf 0-20 %. Unbehandelte jedoch infizierte Kontrollpflanzen wiesen dagegen einen 100%igen Plasmopara-Befall auf.

Beispiel 3.6: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt.

b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24 °C wird der Pilzbefall beurteilt.

Reispflanzen, die mit einer Spritzbrühe behandelt wurden, die als Aktivsubstanz eine Verbindung aus den Tabellen 1 und 2 enthielt, zeigten im Vergleich zu unbehandelten Kontrollpflanzen (100 % Befall) nur geringen Pilzbefall. So reduzierten z.B. im Test (a) die Verbindungen 1.6, 1.8, 1.29 und 1.33 und im Test (b) die Verbindungen 1.6 und 1.33 den Pilzbefall auf 0 bis 20 %.

Beispiel 3.7: Wirkung gegen Pseudomonas tomato an Tomatenpflanzen

a) Tomatenpflanzen werden nach 3-wöchiger Anzucht mit einer aus Spritzpulver des Wirkstoffes hergestellten Spritzbrühe durch Blattapplikation behandelt (Konzentration: 200 ppm). Nach 3,5 Wochen werden die Pflanzen mit einer Bakteriensuspension ($10^8$ Bakterien/ml) inokuliert und für 6 Tage bei hoher Luftfeuchtigkeit und bei einer Temperatur von 25 °C inkubiert. Die Beurteilung der Schutzwirkung erfolgt aufgrund des Bakterienbefalls 7-8 Tage nach Inokulation.

b) Tomatenpflanzen werden nach 3-wöchiger Anzucht mit einer aus Spritzpulver des Wirkstoffes hergestellten Spritzbrühe durch Bodenapplikation behandelt (Konzentration: 60 ppm bezogen auf das Bodenvolumen). Nach 3,5 Wochen werden die Pflanzen mit Bakteriensuspension ($10^8$ Bakterien/ml) inokuliert und für 6 Tage bei hoher Luftfeuchtigkeit und bei einer Temperatur von 25 °C inkubiert.

Die Beurteilung der Schutzwirkung erfolgt aufgrund des Bakterienbefalls 7-8 Tage nach Inokulation.

Verbindungen aus den Tabellen 1 und 2 zeigten gegen Pseudomonas eine gute Schutzwirkung. So verhinderten im Test (a) die Verbindungen 1.1, 1.23, 1.30 und 1.32 und im Test (b) die Verbindungen 1.1, 1.8, 1.23, 1.24, 1.25, 1.27, 1.28, 1.30, 1.32 und 1.34 den Bakterienbefall auf 0 bis 20 %. Unbehandelte jedoch infizierte Kontrollpflanzen wiesen dagegen einen 100%-igen Pseudomonas-Befall auf.

Beispiel 3.8: Wirkung gegen den Tabakmosaik-Virus auf Tabak

8 Wochen alte Tabakpflanzen werden mit einer formulierten Lösung des Wirkstoffes besprüht (Konzentration: 200 ppm) oder injiziert (Konzentration: 200 ppm). Nach 4 Tagen werden die Pflanzen mit einer Suspension des Tabakmosaik-Virus (0.5 $\mu$g/ml + Carborundum) mechanisch inokuliert und bei einer Temperatur von 20 °-22 °C inkubiert.

Die Beurteilung der Schutzwirkung erfolgt aufgrund der Anzahl und Grösse der Lokalläsionen 7 Tage nach der Inokulation.

Verbindungen aus den Tabellen 1 und 2 zeigten eine gute Schutzwirkung gegen den Tabakmosaik-Virus. Infizierte jedoch unbehandelte Kontrollpflanzen wiesen dagegen Läsionen von 100 % auf.

Beispiel 3.9: Wirkung gegen Pseudomonas lachrymans auf Cucumis sativus L.

a) Gurkenpflanzen werden nach 2-wöchiger Anzucht mit einer aus Spritzpulver des Wirkstoffes hergestellten Spritzbrühe besprüht (Konzentration: 20 ppm).

Nach 1 Woche werden die Pflanzen mit einer Bakteriensuspension ($10^8$ Bakterien/ml) infiziert und für 7 Tage bei hoher Luftfeuchtigkeit und einer Temperatur von 23 °C inkubiert.

Die Beurteilung der Schutzwirkung erfolgt aufgrund des Bakterienbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentrationen: 60, 20, 6, 2 ppm bezogen auf das Bodenvolumen).

Nach 1 Woche werden die Pflanzen mit einer Bakteriensuspension ($10^8$ Bakterien/ml) infiziert und für 7 Tage bei hoher Luftfeuchtigkeit und einer Temperatur von 23°C inkubiert.

Die Beurteilung der Schutzwirkung erfolgt aufgrund des Bakterienbefalls 7 - 8 Tage nach der Infektion.

Verbindungen aus den Tabellen 1 und 2 zeigten eine gute Schutzwirkung gegen Pseudomonasbefall. So wurde der Bakterienbefall in Test (a) durch die Verbindungen 1.8 und 1.23. und im Test (b) durch die Verbindung 1.23 auf 0-20 % reduziert. Unbehandelte jedoch infizierte Kontrollpflanzen wiesen dagegen einen 100 %igen Pseudomonas-Befall auf.

Beispiel 3.10: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocken dieses Spritzbelags werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 und 2 zeigten eine gute Schutzwirkung gegen Xanthomonas oryzae. So reduzierten z.B. im Test (a) die Verbindungen 1.1, 1.23 und 1.32 und im Test (b) die Verbindungen 1.1, 1.30, 1.31, 1.32, 1.33, 1.34, 1.35 und 1.36 den Bakterienbefall auf 0 bis 20 %. Unbehandelte jedoch infizierte Kontrollpflanzen wiesen dagegen einen Krankheitsbefall von 100 % auf.

Beispiel 3.11: Wirkung gegen Xanthomonas vesicatoria auf Paprika (Capsicum annuum)

a) Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelages werden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der residualen Wirksamkeit der Prüfsubstanz.

b) Paprikapflanzen der Sorte "California Wonder" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der systemischen Wirksamkeit der Prüfsubstanz.

Verbindungen aus den Tabellen 1 und 2 zeigten eine gute Schutzwirkung gegen Xanthomonas vesicatoria. So reduzierten z.B. im Test (a) die Verbindung 1.32 und im Test (b) die Verbindungen 1.23, 1.30, 1.32, 1.38 und 1.39, den Bakterienbefall auf 0 bis 20 %. Unbehandelte jedoch infizierte Kontrollpflanzen wiesen dagegen einen Krankheitsbefall von 100 % auf.

Beispiel 3.12: Beizwirkung gegen Fusarium nivale an Roggen

Natürlich mit Fusarium nivale infizierter Roggen der Sorte Tetrahell wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Der infizierte und behandelte Roggen wird im Oktober im Freiland mittels einer Sämaschine auf Parzellen von 3 m Länge und 6 Saatreihen ausgesät, mit 3 Wiederholungen pro Versuchsprodukt.

Bis zur Befallsauswertung wird die Versuchspflanzung unter normalen Feldbedingungen kultiviert (vorzugsweise in einer Region mit geschlossener Schneedecke während der Wintermonate).

Zur Ermittlung der Wirkstoffaktivität wird im Frühjahr unmittelbar nach der Schneeschmelze, der prozentuale Anteil mit Fusarium befallener Pflanzen ausgezählt.

Verbindungen aus den Tabellen 1 und 2 zeigten eine gute Schutzwirkung gegen Fusarium. Unbehandelte jedoch infizierte Kontrollpflanzen wiesen einen Krankheitsbefall von 100 % auf.

Beispiel 3.13: Beizwirkung gegen Helminthosporium gramineum an Gerste

Natürlich mit Helminthosporium gramineum infizierte Wintergerste der Sorte "CI" wird auf einer Mischrolle mit der Prüfsubstanz gebeizt, wobei folgende Konzentrationen zur Anwendung gelangen: 600, 200 oder 60 ppm AS (bezogen auf das Gewicht des Saatgutes).

Die infizierte und behandelte Gerste wird im Oktober im Freiland mittels einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität werden zum Zeitpunkt des Aehrenschiebens der prozentuale Anteil mit Helminthosporium befallener Halme ausgezählt.

Verbindungen aus den Tabellen 1 und 2 zeigten eine gute Schutzwirkung gegen Helminthospora. Unbehandelte jedoch infizierte Kontrollpflanzen wiesen einen Krankheitsbefall von 100 % auf.

Beispiel 3.14: Beizwirkung gegen Ustilago nuda an Gerste

Natürlich mit Ustilago nuda infizierte Wintergerste der Sorte "RM1" wird auf einer Mischrolle mit der Prüfsubstanz gebeizt, wobei folgende Konzentrationen zur Anwendung gelangen: 600, 200 oder 60 ppm As (bezogen auf das Gewicht des Saatgutes).

Die infizierte und behandelte Gerste wird im Oktober im Freiland mittels einer Sämaschine auf Parzellen von 2 m Länge und 3 Saatreihen ausgesät, mit 3 Wiederholungen pro Versuchsprodukt.

Bis zur Befallsauswertung wird die Versuchspflanzung unter normalen Feldbedingungen kultiviert.

Zur Ermittlung der Wirkstoffaktivität wird während der Blüte der prozentuale Anteil Ustilago befallener Aehren ausgezählt.

Verbindungen aus den Tabellen 1 und 2 zeigten eine gute Schutzwirkung gegen Ustilago. Unbehandelte jedoch infizierte Kontrollpflanzen wiesen einen Krankheitsbefall von 100 % auf.

Beispiel 3.15: Beizwirkung gegen Colletotrichum lagenarium an Cucumis sativus L.

Gurkensamen werden mit einer Lösung des Wirkstoffes gebeizt (Konzentration: 180 g/100 kg Samen). Die Samen werden ausgesät. Nach 4 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23 °C inkubiert. Die Inkubation wird dann bei normaler Luftfeuchtigkeit und bei 22° bis 23 °C weitergeführt. Die Beurteilung der Schutzwirkung erfolgt aufgrund des Pilzbefalls 7-8 Tage nach der Infektion.

Verbindungen aus den Tabellen 1 und 2 zeigten eine gute Schutzwirkung gegen Colletotrichum. Infizierte Kontrollpflanzen, deren Samen nicht behandelt wurden, wiesen einen Pilzbefall von 100 % auf.

Beispiel 3.16: Residual-protektive Wirkung gegen Venturia inaequalis auf Apfeltrieben

Apfelstecklinge mit 10 - 20 cm langen Frischtrieben werden mit einer aus Spritzpulver des Wirkstoffes hergestellten Spritzbrühe (0,02 % Aktivsubstanz) besprüht. Nach 24 Stunden werden die behandelten Pflanzen mit einer Konidiensuspension des Pilzes infiziert. Die Pflanzen werden dann während 5 Tagen bei 90-100 % relativer Luftfeuchtigkeit inkubiert und während 10 weiteren Tagen in einem Gewächshaus bei 20-24 °C aufgestellt. Der Schorfbefall wird 15 Tage nach der Infektion beurteilt.

Verbindungen aus den Tabellen 1 und 2 zeigten gute Schutzwirkung gegen Venturia. So reduzierte z.B. die Verbindung 1.32 den Schorfbefall auf 5 bis 20 %. Unbehandelte jedoch infizierte Triebe wiesen dagegen einen 100 %igen Venturia-Befall auf.

Beispiel 3.17: Wirkung gegen Erysiphe graminis auf Gerste

a) Ca. 8 cm hohe Gerstenpflanzen werden mit einer aus Spritzpulver des Wirkstoffes hergestellten Spritzbrühe (0,02 % Aktivsubstanz) besprüht. Nach 3 - 4 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

b) Zu ca. 8 cm hohen Gerstenpflanzen wird eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt.

Nach 48 Stunden werden die behandelten Pflanzen mit Konidien des Pilzes bestäubt. Die infizierten Gerstenpflanzen werden in einem Gewächshaus bei ca. 22 °C aufgestellt und der Pilzbefall nach 10 Tagen beurteilt.

Verbindungen aus den Tabellen 1 und 2 zeigten einen guten Schutzeffekt gegen Erysiphe. So reduzierte z.B. die Verbindung 1.24. den Pilzbefall auf weniger als 20 %, während unbehandelte aber infizierte Kontrollpflanzen zu 100 % befallen waren.

## Patentansprüche

## Patentansprüche für folgende Vertragsstaaten : CH, DE, FR, GB, IT, LI, NL

**1.** Verbindungen der Formel I

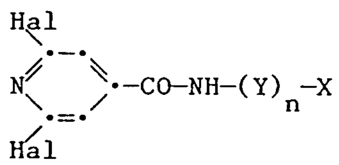

(I)

in welcher bedeuten:

Hal unabhängig voneinander Fluor, Chlor, Brom oder Jod;
Y NH;
n 1 oder 0;
X ein 5-gliedriger gesättigter oder ungesättigter unsubstituierter oder mit $C_1$-$C_8$-Alkyl, Halogen, Trifluormethyl, Cyano oder Nitro substituierter Heterocyclus mit 1-3 Heteroatomen, wie N, O oder S, wobei der Heterocyclus zusätzlich eine Oxo- oder Thionogruppe enthalten kann.

**2.** Verbindungen gemäss Anspruch 1 der Formel Ib

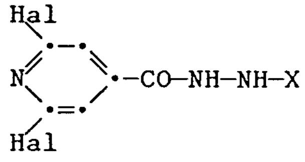

(Ib)

worin bedeuten:

Hal unabhängig voneinander Fluor, Chlor oder Brom;
X ein wie unter Formel I angegebener Heterocyclus.

3. Verbindungen gemäss Anspruch 1 der Formel Ic

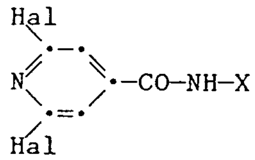

(Ic)

worin bedeuten:

Hal unabhängig voneinander Fluor, Chlor oder Brom;

X ein wie unter Formel I angegebener Heterocyclus.

4. Verbindungen gemäss Anspruch 3, worin bedeuten:

Hal Chlor oder Brom;

X Furan-2-yl, 1,2,4-Triazol-3-yl, Thiazol-2-yl, Thiazol-5-yl, Isothiazol-3-yl, Oxazol-2-yl, Isoxazol-3-yl, Isoxazol-5-yl, 1,3,4-Thiadiazol-2-yl, 1,2,4-Thiadiazol-3-yl oder 3-Thiono-1,2-dithia-4-azol-5-yl.

5. Eine Verbindung aus der Gruppe:
3-Methylisoxazol-5-(2,6-dichlorisonicotinsäure)-amid,
5-Methylisoxazol-3-(2,6-dichlorisonicotinsäure)-amid,
α-Methylfurfuryl-2,6-dichlorisonicotinsäureamid;
3-Methylisothiazol-5-(2,6-dichlorisonicotinsäure)-amid.

6. Verfahren zur Herstellung der Verbindungen der Formel I in Anspruch 1, dadurch gekennzeichnet, dass man ein Isonicotinsäurehalogenid der Formel II

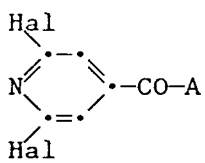

(II)

mit einem Amin der Formel III

$H_2N-(Y)_n-X$ (III)

in Gegenwart einer Base in einem inerten Lösungsmittel oder ohne Lösungsmittel bei Temperaturen von -20° bis 150°C, vorzugsweise 0° bis 80°C, umsetzt, wobei Hal Halogen, A Halogen, einen Acyloxyrest der Formel

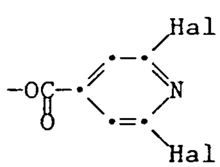

,

Imidazolyl oder Triazolyl bedeuten und X, Y und n die unter der Formel I angegebenen Bedeutungen besitzen.

7. Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**8.** Mittel gemäss Anspruch 9, dadurch gekennzeichnet, dass es als aktive Komponente mindestens eine Verbindung gemäss den Ansprüchen 2-4 enthält.

**9.** Mittel gemäss Anspruch 9, dadurch gekennzeichnet, dass es als aktive Komponente mindestens eine Verbindung der Formel I gemäss Anspruch 5 enthält.

**10.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**11.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2-5 auf die Pflanze oder deren Standort appliziert.

**12.** Verwendung von Verbindungen gemäss Anspruch 1 zum Schutz von Pflanzen gegen den Befall durch phytopathogene Mikroorganismen.

**13.** Verwendung von Verbindungen gemäss einem der Ansprüche 2-5 zum Schutz von Pflanzen gegen den Befall durch phytopathogene Mikroorganismen.

**Patentansprüche für folgenden Vertragsstaat : ES**

**1.** Verfahren zur Herstellung der Verbindungen der Formel I

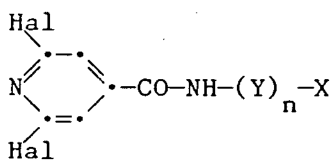

(I)

in welcher bedeuten:

Hal unabhängig voneinander Fluor, Chlor, Brom oder Jod;

Y NH;

n 1 oder 0;

X ein 5-gliedriger gesättigter oder ungesättigter unsubstituierter oder mit $C_1$-$C_8$-Alkyl, Halogen, Trifluormethyl, Cyano oder Nitro substituierter Heterocyclus mit 1-3 Heteroatomen, wie N, O oder S, wobei der Heterocyclus zusätzlich eine Oxo- oder Thionogruppe enthalten kann, dadurch gekennzeichnet, dass man ein Isonicotinsäurehalogenid der Formel II

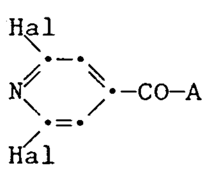

(II)

mit einem Amin der Formel III

$H_2N$-$(Y)_n$-X (III)

in Gegenwart einer Base in einem inerten Lösungsmittel oder ohne Lösungsmittel bei Temperaturen von -20° bis 150°C, vorzugsweise 0° bis 80°C, umsetzt, wobei Hal Halogen, A Halogen, einen Acyloxyrest der Formel

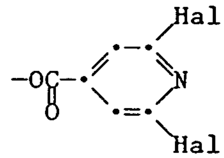 ,

Imidazolyl oder Triazolyl bedeuten und X, Y und n die unter der Formel I angegebenen Bedeutungen besitzen.

**2.** Verfahren gemäss Anspruch 1, dadurch gekennzeichnet, dass Verbindungen der Formel 1b

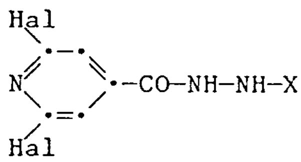 (Ib)

worin bedeuten:

Hal unabhängig voneinander Fluor, Chlor oder Brom;

X ein wie unter Formel I in Anspruch 1 angegebener Heterocyclus; hergestellt werden.

**3.** Verfahren gemäss Anspruch 1, dadurch gekennzeichnet, dass Verbindungen der Formel Ic

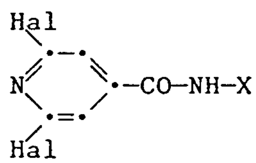 (Ic)

worin bedeuten:

Hal unabhängig voneinander Fluor, Chlor oder Brom;

X ein wie unter Formel I in Anspruch 1 angegebener Heterocyclus; hergestellt werden.

**4.** Verfahren gemäss Anspruch 1, dadurch gekennzeichnet, dass Verbindungen der Formel Ib in Anspruch 2, worin bedeuten:

Hal Chlor oder Brom;

X Furan-2-yl, 1,2,4-Triazol-3-yl, Thiazol-2-yl, Thiazol-5-yl, Isothiazol-3-yl, Oxazol-2-yl, Isoxazol-3-yl, Isoxazol-5-yl, 1,3,4-Thiadiazol-2-yl, 1,2,4-Thiadiazol-3-yl oder 3-Thiono-1,2-dithia-4-azol-5-yl; hergestellt werden.

**5.** Verfahren gemäss Anspruch 1, dadurch gekennzeichnet, dass eine Verbindung aus der Gruppe:
3-Methylisoxazol-5-(2,6-dichlorisonicotinsäure)-amid,
5-Methylisoxazol-3-(2,6-dichlorisonicotinsäure)-amid,
α-Methylfurfuryl-2,6-dichlorisonicotinsäureamid,
3-Methylisothiazol-5-(2,6-dichlorisonicotinsäure)-amid,
hergestellt wird.

**Claims**

**Claims for the following Contracting States : CH, DE, FR, GB, IT, LI, NL**

**1.** A compound of formula I

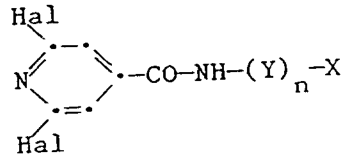

(I)

in which:
Hal, each independently of the other, is fluorine, chlorine, bromine or iodine;
Y is NH;
n is 1 or 0;
X is a 5-membered saturated or unsaturated, unsubstituted or $C_1$-$C_8$alkyl-, halogen-, trifluoromethyl-, cyano- or nitro-substituted heterocycle having from 1 to 3 hetero atoms, such as N, O or S, wherein the heterocycle may, in addition, contain an oxo or thiono group.

**2.** A compound according to claim 1 of formula Ib

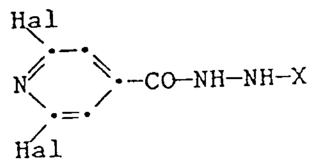

(Ib)

in which:
Hal, each independently of the other, is fluorine, chlorine or bromine;
X is a heterocycle as specified for formula I.

**3.** A compound according to claim 1 of formula Ic

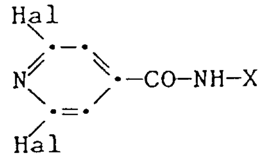

(Ic)

in which:
Hal, each independently of the other, is fluorine, chlorine or bromine;
X is a heterocycle as specified for formula I.

**4.** A compound according to claim 3 in which:
Hal is chlorine or bromine;
X is furan-2-yl, 1,2,4-triazol-3-yl, thiazol-2-yl, thiazol-5-yl, isothiazol-3-yl, oxazol-2-yl, isoxazol-3-yl, isoxazol-5-yl, 1,3,4-thiadiazol-2-yl, 1,2,4-thiadiazol-3-yl or 3-thiono-1,2-dithia-4-azol-5-yl.

**5.** A compound from the group:
3-methylisoxazole-5-(2,6-dichloroisonicotinic acid) amide,
5-methylisoxazole-3-(2,6-dichloroisonicotinic acid) amide,
α-methylfurfuryl-2,6-dichloroisonicotinic acid amide and
3-methylisothiazole-5-(2,6-dichloroisonicotinic acid) amide.

6. A process for the preparation of a compound of formula I in claim 1, which comprises reacting an isonicotinic acid halide of formula II

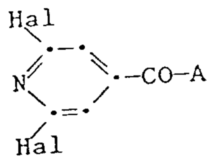

(II)

with an amine of formula III

$H_2N-(Y)_n-X$ (III)

in the presence of a base in an inert solvent or without a solvent at temperatures of from -20° to 150°C, preferably from 0° to 80°C, wherein Hal is halogen, A is halogen, an acyloxy radical of the formula

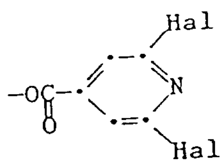

,

imidazolyl or triazolyl, and X, Y and n are as defined for formula I.

7. A composition for protecting plants against attack by microorganisms that comprises as active component at least one compound according to claim 1 together with customary carriers and adjuvants.

8. A composition according to claim 9 that comprises as active component at least one compound according to claims 2 to 4.

9. A composition according to claim 9 that comprises as active component at least one compound of formula I according to claim 5.

10. A method of protecting plants against attack by phytopathogenic microorganisms which comprises applying as active ingredient to the plant or the locus thereof a compound according to claim 1.

11. A method of protecting plants against attack by phytopathogenic microorganisms which comprises applying as active ingredient to the plant or the locus thereof a compound according to any one of claims 2 to 5.

12. The use of a compound according to claim 1 for protecting plants against attack by phytopathogenic microorganisms.

13. The use of a compound according to any one of claims 2 to 5 for protecting plants against attack by phytopathogenic microorganisms.

**Claims for the following Contracting State : ES**

**1.** A process for the preparation of a compound of formula I

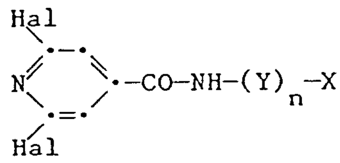

(I)

in which:
Hal, each independently of the other, is fluorine, chlorine, bromine or iodine;
Y is NH;
n is 1 or 0;
X is a 5-membered saturated or unsaturated, unsubstituted or $C_1$-$C_8$alkyl-, halogen-, trifluoromethyl-, cyano- or nitro-substituted heterocycle having from 1 to 3 hetero atoms, such as N, O or S, wherein the heterocycle may, in addition, contain an oxo or thiono group, which comprises reacting an isonicotinic acid halide of formula II

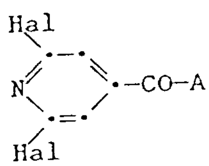

(II)

with an amine of formula III

$H_2N$-$(Y)_n$-X (III)

in the presence of a base in an inert solvent or without a solvent at temperatures of from -20° to 150°C, preferably from 0° to 80°C, wherein Hal is halogen, A is halogen, an acyloxy radical of the formula

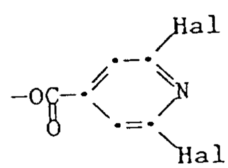

,

imidazolyl or triazolyl, and X, Y and n are as defined for formula I.

**2.** A process according to claim 1 wherein a compound of formula Ib

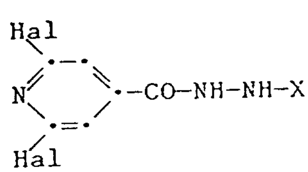

(Ib)

in which:
Hal, each independently of the other, is fluorine, chlorine or bromine;
X is a heterocycle as specified for formula I in claim 1, is prepared.

**3.** A process according to claim 1 wherein a compound of formula Ic

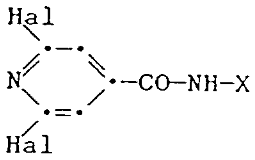

(Ic)

in which:
Hal, each independently of the other, is fluorine, chlorine or bromine;
X is a heterocycle as specified for formula I in claim 1, is prepared.

**4.** A process according to claim 1 wherein a compound of formula Ib in claim 2 is prepared in which:
Hal is chlorine or bromine;
X is furan-2-yl, 1,2,4-triazol-3-yl, thiazol-2-yl, thiazol-5-yl, isothiazol-3-yl, oxazol-2-yl, isoxazol-3-yl, isoxazol-5-yl, 1,3,4-thiadiazol-2-yl, 1,2,4-thiadiazol-3-yl or 3-thiono-1,2-dithia-4-azol-5-yl.

**5.** A process according to claim 1 wherein a compound from the group:
3-methylisoxazole-5-(2,6-dichloroisonicotinic acid) amide,
5-methylisoxazole-3-(2,6-dichloroisonicotinic acid) amide,
α-methylfurfuryl-2,6-dichloroisonicotinic acid amide and
3-methylisothiazole-5-(2,6-dichloroisonicotinic acid) amide
is prepared.

**Revendications**
**Revendications pour les Etats contractants suivants : CH, DE, FR, GB, IT, LI, NL**

**1.** Composés de formule I

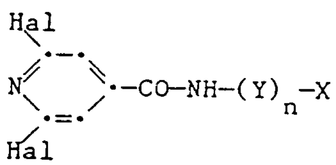

(I)

dans laquelle

| | |
|---|---|
| les symboles Hal | représentent chacun, indépendamment l'un de l'autre, le fluor, le chlore, le brome ou l'iode; |
| Y | représente NH; |
| n | est égal à 1 ou 0; |
| X | représente un hétérocycle saturé ou insaturé de 5 chaînons, non substitué ou substitué par des groupes alkyle en C 1-C 8, des halogènes, des groupes trifluorométhyle, cyano ou nitro et contenant 1 à 3 hétéroatomes tels que N, O ou S, cet hétérocycle pouvant en outre contenir un groupe oxo ou thiono. |

**2.** Composés selon revendication 1, de formule Ib

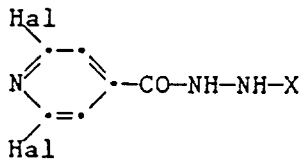

(Ib)

dans laquelle

les symboles Hal représentent chacun, indépendamment l'un de l'autre, le fluor, le chlore ou le brome;

X représente un hétérocycle tel que défini en référence à la formule I.

**3.** Composés selon revendication 1, de formule Ic

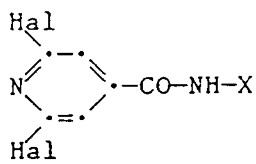

(Ic)

dans laquelle

les symboles Hal représentent chacun, indépendamment l'un de l'autre, le fluor, le chlore ou le brome;

X représente un hétérocycle tel que défini en référence à la formule I.

**4.** Composés selon revendication 3, pour lesquels
Hal représente le chlore ou le brome;
X représente un groupe furanne-2-yle, 1,2,4-triazole-3-yle, thiazole-2-yle, thiazole-5-yle, isothiazole-3-yle, oxazole-2-yle, isoxazole-3-yle, isoxazole-5-yle, 1,3,4-thiadiazole-2-yle, 1,2,4-thiadiazole-3-yle ou 3-thiono-1,2-dithia-4-azole-5-yle.

**5.** Un composé du groupe suivant :
3-méthylisoxazole-5-(2,6-dichloroisonicotinamide),
5-méthylisoxazole-3-(2,6-dichlorisonicotinamide),
α-méthylfurfuryl-2,6-dichlorisonicotinamide;
3-méthylisothiazole-5-(2,6-dichlorisonicotinamide).

**6.** Procédé de préparation des composés de formule I de la revendication 1, caractérisé en ce que l'on fait réagir un halogénure d'acide isonicotinique de formule II

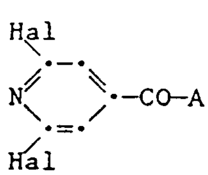

(II)

avec une amine de formule III

$H_2N\text{-}(Y)_n\text{-}X$ (III)

en présence d'une base, dans un solvant inerte ou sans solvant, à des températures de -20 à 150°C,

de préférence de 0 à 80 °C, Hal représentant un halogène, A un halogène, un groupe acyloxy de formule

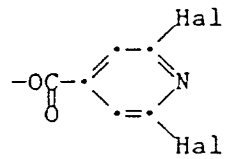 ,

un groupe imidazolyle ou triazolyle et X, Y et n ayant les significations indiquées en référence à la formule I.

**7.** Produit pour protéger les végétaux contre l'attaque par des microorganismes, caractérisé en ce qu'il contient au moins un composant actif consistant en un composé selon revendication 1, avec des véhicules et produits auxiliaires usuels.

**8.** Produit selon revendication 7, caractérisé en ce qu'il contient au moins un composant actif consistant en un composé selon les revendications 2 à 4.

**9.** Produit selon revendication 7, caractérisé en ce qu'il contient au moins un composant actif consistant en un composé de formule I de la revendication 5.

**10.** Procédé pour protéger les végétaux contre l'attaque par des microorganismes phytopathogènes, caractérisé en ce que l'on applique sur la plante ou son habitat une substance active consistant en un composé selon revendication 1.

**11.** Procédé pour protéger les végétaux contre l'attaque par des microorganismes phytopathogènes, caractérisé en ce que l'on applique sur la plante ou son habitat une substance active consistant en un composé selon l'une des revendications 2 à 5.

**12.** Utilisation des composés selon revendication 1 pour la protection des végétaux contre l'attaque par des microorganismes phytopathogènes.

**13.** Utilisation des composés selon l'une des revendications 2 à 5 pour la protection des végétaux contre l'attaque par des microorganismes phytopathogènes.

**Revendications pour l'Etat contractant suivant : ES**

**1.** Procédé de préparation des composés de formule I

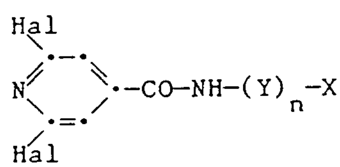 (I)

dans laquelle

| | |
|---|---|
| les symboles Hal | représentent chacun, indépendamment l'un de l'autre, le fluor, le chlore, le brome ou l'iode; |
| Y | représente NH; |
| n | est égal à 1 ou 0; |

X représente un hétérocycle de 5 chaînons, saturé ou insaturé, non substitué ou substitué par des groupes alkyle en C 1-C 8, des halogènes, des groupes trifluorométhyle, cyano ou nitro, et contenant de 1 à 3 hétéroatomes tels que N, O ou S, cet hétérocycle pouvant contenir en outre un groupe oxo ou

thiono, caractérisé en ce que l'on fait réagir un halogénure d'acide isonicotinique de formule II

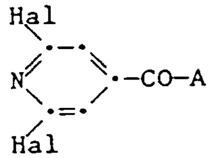

(II)

avec une amine de formule III

$H_2N-(Y)_n-X$ (III)

en présence d'une base, dans un solvant inerte ou sans solvant, à des températures de -20 à 150 °C, de préférence de 0 à 80 °C, Hal représentant un halogène, A un halogène, un groupe acyloxy de formule

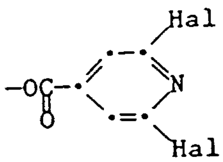

,

un groupe imidazolyle ou triazolyle et X, Y et n ayant les significations indiquées en référence à la formule I.

**2.** Procédé selon revendication 1, caractérisé en ce que l'on prépare des composés de formule Ib

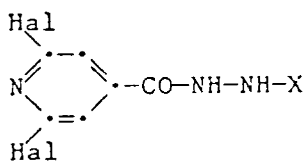

(Ib)

dans laquelle
les symboles Hal représentent chacun, indépendamment l'un de l'autre, le fluor, le chlore ou le brome;
X représente un hétérocycle tel que défini en référence à la formule I dans la revendication 1.

**3.** Procédé selon revendication 1, caractérisé en ce que l'on prépare des composés de formule Ic

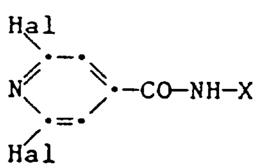

(Ic)

dans laquelle
les symboles Hal représentent chacun, indépendamment l'un de l'autre, le fluor, le chlore ou le brome;
X représente un hétérocycle tel que défini en référence à la formule I dans la revendication 1.

**4.** Procédé selon revendication 1, caractérisé en ce que l'on prépare des composés de formule Ib de la revendication 2, dans laquelle
Hal représente le chlore ou le brome;
X représente un groupe furanne-2-yle, 1,2,4-triazole-3-yle, thiazole-2-yle, thiazole-5-yle, isothiazole-3-yle, oxazole-2-yle, isoxazole-3-yle, isoxazole-5-yle, 1,3,4-thiadiazole-2-yle, 1,2,4-thiadiazole-3-yle ou 3-thiono-1,2-dithia-4-azole-5-yle.

**5.** Procédé selon revendication 1, caractérisé en ce que l'on prépare un composé du groupe suivant :
3-méthylisoxazole-5-(2,6-dichlorisonicotinamide);
5-méthylisoxazole-3-(2,6-dichlorisonicotinamide);
$\alpha$-méthylfurfuryl-2,6-dichlorisonicotinamide;
3-méthylisothiazole-5-(2,6-dichlorisonicotinamide).